(19) Europäisches Patentamt
European Patent Office
Office européen des brevets

(11) EP 0 887 348 A1

(12) EUROPEAN PATENT APPLICATION

(43) Date of publication:
30.12.1998 Bulletin 1998/53

(21) Application number: 97110336.1

(22) Date of filing: 25.06.1997

(51) Int. Cl.[6]: C07D 401/14, A61K 31/40, C07D 409/14, C07D 403/14, C07D 405/14, C07D 209/10, C07F 9/38

(84) Designated Contracting States:
AT BE CH DE DK ES FI FR GB GR IE IT LI LU MC NL PT SE

(71) Applicant:
Boehringer Mannheim Italia S.p.A.
20052 Monza (MI) (IT)

(72) Inventor:
The designation of the inventor has not yet been filed

(74) Representative:
Weber, Manfred, Dr. et al
c/o Boehringer Mannheim GmbH,
Patentabteilung,
Sandhoferstrasse 116
68298 Mannheim (DE)

# (54) Bis-Indole derivatives having antimetastatic activity, a process for their preparation and pharmaceutical compositions containing them

(57) The present invention relates to compounds of the general formula (I):

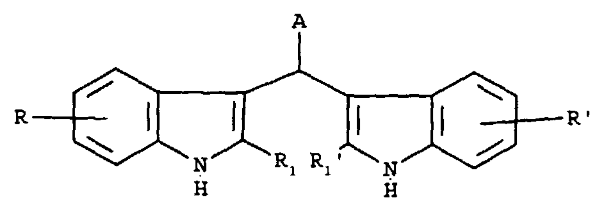

(I)

wherein:

- R and R' are independently selected from hydrogen, hydroxy, chlorine, bromine, iodine, fluorine, $(C_1-C_6)$alkyl, $(C_1-C_4)$alkoxy, $(C_1-C_4)$acyloxy, amino, mono-$(C_1-C_4)$alkylamino, di-$(C_1-C_4)$alkylamino, -SH, $(C_1-C_4)$alkylthio, carboxy, $(C_1-C_4)$alkoxycarbonyl groups;
- $R_1$ and $R_1$' are independently selected from hydrogen, hydroxy, hydroxymethyl, amino, carboxy, $(C_1-C_4)$alkyl groups;
- A is a
  - phenyl or naphtyl group substituted by at least one group selected from hydoxy, carboxy, -SH, -CONHOH or -$PO_3H_2$ group, or is a
  - 5- or 6-membered heterocycle containing one or two heteroatoms selected from oxygen, nitrogen or sulfur, which can be optionally benzocondensed and/or substituted by at least one group selected from pyridyl, -SH, -$PO_3H_2$, carboxy or
- CONHOH, or is a
  - group of formula $-(CH_2)_n-X$, in which n is zero or the integers 1 or 2, and X is selected from -SH -$PO_3H_2$, -CONHOH, carboxy, amino, mono-$(C_1-C_4)$alkylamino, di-$(C_1-C_4)$alkylamino, enantiomers, diastereoisomers, racemates and mixtures thereof, as well as salts thereof with pharmaceutically acceptable acids and bases, as antitumor and antimetastatic agents.

EP 0 887 348 A1

## Description

The present invention relates to bis-indole derivatives and to their use to inhibit metastatic processes.

### BACKGROUND OF THE INVENTION

In the past the therapy of the tumors has been achieved by surgical intervention, radiation treatment and chemotherapy. The drawbacks of this latter are mainly due to the toxicity of the cytotoxic drugs, which is usually not restricted to the cancer cells, and to the acquired resistance of the cancer cells to some of the most widely used drugs, which vanifies the final result of the therapy.

On the other hand, the elimination of the primary tumor by surgery is not always possible and in any case does not prevent the most metastasizing tumors, such as for example breast cancer or melanoma, to invade other target organs, which develop further secondary tumors after months or years from the surgical treatment. These secondary tumors are usually the main cause of death of the patient.

In the years it has become apparent that the therapy of the metastasizing tumors is unlikely to bring to the complete cure of the patient; therefore, the treatment with cytotoxic drugs is now seen as a palliative and life-prolonging method rather than a curative method. A cronical treatment with a drug having low toxicity would be preferable while targeted to the control of the progression of the disease. An example of such therapy is the treatment of invasive breast cancer with tamoxifen.

The efforts of many researchers have been focused recently to the development of drugs able to inhibit the invasive process of the tumor which brings to metastases' formation.

Several mechanisms have been proposed and several drugs have been tested both in "in vitro" and "in vivo" models. As an example, styryl diphenylamine derivatives were found to revert the transformed phenotype of human fibrosarcoma HT1080 cells. These compounds can inhibit metastasis in mice with negligible toxicity [Anticancer Res., 17(1A), 393-400 (1997)].

Other known molecules, such as the stable prostacyclin analog cicaprost and docosahexaaenoic acid, were shown to have antimetastatic properties in "in vivo" models [Cancer Detect. Prev., 21(1), 44-50 (1997) and Br. J. Cancer, 75(5), 650-655 (1997), respectively].

Peptides (and their poly-ethylene glycol hybrids) related to the core sequence of the type III connecting segment domain of fibronectin A have inhibitory effect on the experimental metastasis of B16-BL6 melanoma [Biol. Pharm. Bull., 19(12), 1574-1579 (1996)].

The effect of low molecular weight glycoamine analogues on the metastasis of MDA-MB-435 human breast carcinoma xenografts growing in the mammary fat pads of nude mice was also reported [Cancer Res., 56(23), 5319-5324 (1996)]. The proposed mechanism of synthetic glycoamines may include the inhibition of beta-galectin-mediated homotypic cancer cell aggregation and induction of apoptosis in target cells.

The antimetabolite tiazofurin was described to possess inhibitory effects on the metastatization of HT 168-M1 human melanoma cell line, due to inhibition of adhesion and migration of tumor cells [Anticancer Res., 16(6A), 3307-3312 (1996)].

In J. Immunother. Emphasis Tumor Immunol., 19(5), 324-333 (1996) it is also reported that the immunoregulatory octapeptide THF-gamma 2 is able to inhibit murine Lewis lung carcinoma metastases via intranasal administration.

The angiogenesis inhibitor TNP-470 is active in the treatment of hepatic metastases in rats following intraportal implantation of rat ascites hepatoma AH-130 cells [J. Surg. Res., 64(1), 35-41 (1996)].

Other compounds which showed antimetastatic effect are the novel indolocarbazole NB-506 [Jpn. J. Cancer Res., 87(5), 518-523 (1996)] and some structurally related analogues if nojirimycin A [J. Antibiot. (Tokyo), 49(2), 155-161 (1996)].

Among the targets that have been evaluated up to now to give rise to a possible antimetastatic activity, the inhibition of the matrix metallo-proteinases seems to be one of the most promising.

The matrix metallo-proteinases (or metallo-proteases), which are upregulated in the cancer cells, degrade the extracellular matrix and bring to the propagation of the tumor cells into the blood stream to reach the target organs where the metastasis develop.

Nevertheless, since different types of such proteases exist in the organism and are implicated in the regulation of vital functions, a high selectivity is desired, in order to avoid the toxic side effects, especially in a chronical treatment.

A number of compounds are known in the literature [see review article of Nigel R.A. Beeley et al., Curt. Opin. Ther. Patents, 4(1), 7 (1994)] or are descibed in the patent literature [WO-A-92/09563 by Glycomed, EP-A-497 192 by Hoffmann-LaRoche, WO-A-90/05719 by British Biotechnology, EP-A-489 577 by Celltech, EP-A-320 118 by Beecham, US-A-4,595,700 by Searle]. In particular, batimastat and marimastat have been developed by British Biotechnology and the latter is now under investigation in clinical trials. However, such compounds are broad inhibitors of matrix metalloproteinases, therefore therapy with these molecules might be associated to undesirable toxicity.

It is therefore evident that there is still a high need of new compounds, which must have a low toxicity and a marked activity in inhibiting both the tumor growth and the metastasis process, as candidates for a chronical antitumor therapy.

We have found that a class of bis-indole derivatives possess the requisites essential for such antitumor and antimetastatic activity.

Bis-indoles linked by a pyridylmethane diradical were described in U.S. 3,409,626 as intermediates for the synthesis of pyridinium salts having antihypertensive activity. Other pyridyl derivatives were also disclosed in J. Org. Chem., 41(5), 870-875 (1976).

Other bis-indoles which conform with the formula (I) showed below and having as A group an amino methyl, carboxy, methoxycarbonyl or alkoxycarbonylmethyl group are also known from J. of Natural Products, 58(8), 1254-60 (1995), J. of Natural Products, 54(2), 564-9 (1991), Synthesis, 872 (1984), Tetrahedron, 44(18), 5897-5904 (1988).

Bis-indole hydroxyphenylmethanes have been reported in Nat. Prod. Lett., 4(4), 309-312 (1994) (4-hydroxyphenyl, as antimicrobial agent) and in J. Het. Chem., 14(8), 1361-1363 (1977) (3,4-dihydroxyphenyl), Pakistan J. Sci. Ind. Res., 14(1-2), 15-18 (1971) (2-hydroxyphenyl), Indian J. Chem., Sect. B, 25B(12), 1204-1208 (1986) (2-hydroxyphenyl and methyl in position 2 of the indole rings), while bis-indole heteroaryl methanes have been reported in Tetrahedron, 47(44), 9225-9230 (1991) (heteroaryl = 2-furanyl, 2-thienyl).

No antitumor or antimetastatic activity has been reported so far for this class of compounds.

DESCRIPTION OF THE INVENTION

The present invention relates to the use of bis-indole derivatives of formula (I):

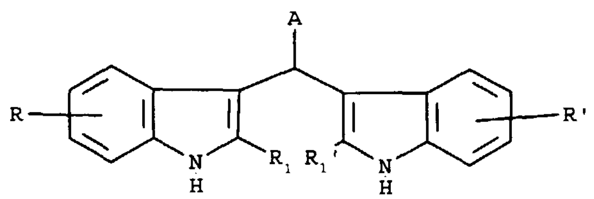

(I)

wherein:

- R and R' are independently selected from hydrogen, hydroxy, chlorine, bromine, iodine, fluorine, $(C_1-C_6)$alkyl, $(C_1-C_4)$alkoxy, $(C_1-C_4)$acyloxy, amino, mono-$(C_1-C_4)$alkylamino, di-$(C_1-C_4)$alkylamino, -SH, $(C_1-C_4)$alkylthio, carboxy, $(C_1-C_4)$alkoxycarbonyl groups;
- $R_1$ and $R_1$' are independently selected from hydrogen, hydroxy, hydroxymethyl, amino, carboxy, $(C_1-C_4)$alkyl groups;
- A is a
  - phenyl or naphtyl group substituted by at least one group selected from hydoxy, carboxy, -SH, -CONHOH or -$PO_3H_2$ group, or is a
  - 5- or 6-membered heterocycle containing one or two heteroatoms selected from oxygen, nitrogen or sulfur, which can be optionally benzocondensed and/or substituted by at least one group selected from pyridyl, -SH, -$PO_3H_2$, carboxy or
- CONHOH, or is a
  - group of formula $-(CH_2)_n-X$, in which n is zero or the integers 1 or 2, and X is selected from -SH, -$PO_3H_2$, -CONHOH, carboxy, amino, mono-$(C_1-C_4)$alkylamino, di-$(C_1-C_4)$alkylamino, enantiomers, diastereoisomers, racemates and mixtures thereof, as well as salts thereof with pharmaceutically acceptable acids and bases, as antitumor and antimetastatic agents.

The present invention also relates to the new compounds of formula (I) and to a process for obtaining them, with the proviso that the following known compounds are excluded:

- compounds in which R, R', $R_1$, $R_1$' are hydrogen and A is pyridyl 2-furanyl, 2-thienyl, carboxy, methoxycarbonyl, $(C_1-C_4)$alkoxycarbonylmethyl, 2- or 4-hydroxyphenyl 3,4-dihydroxyphenyl;
- $R_1$, $R_1$' are hydrogen, R and R' are 6-bromo and A is a aminomethyl group;

- $R_1$, $R_1$' are hydrogen, R and R' are 5-chloro, 5-bromo, 5-carboxy or 5-methoxy and A is a 4-pyridyl group;
- R, R' are hydrogen, $R_1$ and $R_1$' are methyl and A is 2-furanyl, 2-thienyl or 2-hydroxyphenyl.

Another object of the present invention is to provide pharmaceutical compositions containing an effective dosage of at least one compound of formula (I) in admixture with suitable excipients or diluents.

When A is an heterocycle, it is preferably selected from pyridyl, imidazol-2-yl, thienyl, quinolinyl, isoquinolinyl, pyrrolyl, furanyl, 5-methylfuran-2-yl, 5-hydroxymethylfuran-2-yl, 8-hydroxyquinolin-2-yl, dipyridyl.

When A is a substituted phenyl group it is preferably selected from 2-carboxyphenyl, 3-carboxy-4-hydroxyphenyl, 4-methoxycarbonylphenyl, 3,4-dihydroxyphenyl, 2,3-dihydroxyphenyl, 3-carboxyphenyl, 4-carboxyphenyl.

Preferred compounds of formula (I) are those in which $R_1$ and $R_1$' and/or R and R' are hydrogen.

Other preferred compounds of formula (I) are those in which A is a 2-,3- or 4-pyridyl group.

Particularly preferred compounds are those in which $R_1$, $R_1$' are hydrogen, R and R' are hydrogen or a hydroxy group and A is a pyridyl group.

The most preferred compounds are 3-[bis(indol-3-yl)methyl]pyridine and 3-[bis(5-hydroxyindol-3-yl)methyl]pyridine.

PREPARATION OF THE COMPOUNDS OF THE INVENTION

The symmetrical compounds of formula (I) in which R = R' and $R_1$ = $R_1$' can be prepared by means of the condensation of an aldehyde of formula (II):

$$A\text{-}CHO \qquad (II)$$

with at least two equivalents of an indole of formula (III):

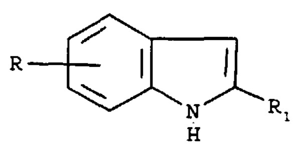

(III)

wherein R, $R_1$ and A have the above meanings. Such a reaction can be performed in a solvent, preferentially an alcohol, water or mixtures thereof and in the presence of an acidic catalyst. Suitable acids are organic acids, preferably acetic acid, inorganic acids, preferably hydrochloric acid or Lewis acids. When acetic acid is used, it is also used as the solvent. The reaction temperature can vary from - 10°C to 100°C, but it is generally kept between 0°C and room temperature.

When the starting indole derivative is activated to the electrophilic reactions, the acid catalysis is not required.

This condensation can also be performed in neutral ambient, by photochemical activation as described in Tetrahedron, 47(44), 9225-9230 (1991), which is herein incorporated by reference.

When A is an alkoxycarbonylmethyl residue, a best reaction is to condensate two equivalents of the intermediate of formula (III) with propiolic acid alkyl ester, as described in J. Chinese Chem Soc., 21, 229-234 (1974), which is herein incorporated by reference.

The unsymmetrical compounds of formula (I) in which R and $R_1$ are different from R' and $R_1$', respectively, can be performed by a process which encompasses the following steps:

(a) Friedels-Craft reaction of a compound of formula (III) with an intermediate of formula (IV):

$$A\text{-}CO\text{-}Hal \qquad (IV)$$

in which A is as above described and Hal is chlorine, bromine or iodine, in a solvent and in the presence of a Lewis acid, preferably $AlCl_3$;

(b) Horner-Emmonds reaction of the product of step (a) with a compound of formula (V):

$$(AlkO)_2PO\text{-}CH_2\text{-}COOAlk \qquad (V)$$

in which Alk is a lower alkyl group, in the presence of a strong base such as sodium hydride and in an inert solvent such as tetrahydrofuran, to give a compound of formula (VI):

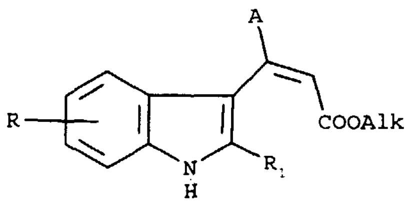

(VI)

(c) reduction of the C=C double bond, for example by means of catalytic hydrogenation, followed by the reduction of the -COOAlk to aldehyde, for example by means of di-isobutyl aluminium hydride at temperatures ranging from -70°C to 0°C, preferably between -70°C and -30°C, and condensation of the resulting product with one equivalent of a hydrazine of formula (VII):

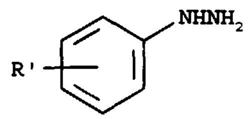

(VII)

(d) formation of the second indole ring by means of a Fischer reaction, to give compounds of formula (I) in which $R_1$' is hydrogen.

Compounds in which $R_1$' is different from hydrogen can be obtained either by electrophilic addition to the 2-position of the unsubstituted derivative [J. Chem. Soc., Perkin Trans., 1, 2543-2551 (1987), which is herein incorporated by reference] or by a modification of the above Fischer synthesis, in which the -COOAlk substituent of intermediate (VI) is transformed into a ketone substituent -CO-$R_1$' (for example, by reaction with alkyl lithium compounds) and it is then reacted as indicated above.

Optically pure compounds of formula (I) can be obtained by means of the usual methods to separate isomers and diastereoisomers, such as optical resolution, by crystallizing diastereoisomeric salts of compounds of formula (I) with optically active acids or bases, or chromatographic separation of diastereoisomers or diastereoisomeric salts of compounds of formula (I). Examples of suitable optically active acids are D- or L-tartaric acid, mandelic acid, malic acid, lactic acid or camphorsulfonic acid. Examples of suitable optically active bases are D- or L-$\alpha$-phenylethylamine, ephedrine, quinidine or cinchonidine.

Alkaline salts, ammonium salts, acetates or hydrochlorides are mainly used as pharmacologically acceptable salts which are produced in the usual manner, e.g. by titrating the compounds with inorganic or organic bases or inorganic acids such as sodium hydroxide, potassium hydroxide, aqueous ammonia, amines such as triethylamine or hydrochloric acid. The salts are usually purified by reprecipitation from suitable solvents.

The intermediates of formula (II) are commercial products or can be obtained by means of conventional reactions which are part of the general knowledge of the skilled man, starting from precursors in which the aldehyde group is preferentially protected (for example by means of an acetal protecting group).

For example, when A carries a carboxy functionality (such as in glyoxylic acid), the hydroxamic acid derivative can be obtained by reacting the -COOH with O-benzylhydroxylamine in the presence of a condensing agent (such as dicyclohexyl carbodiimide), followed by the hydrogenolysis to eliminate the benzyl group.

When A carries a hydroxy group, it can be submitted to the following reactions:

- conversion of the -OH group into a halogen group (for example into a chlorine group by means of thionyl chloride), followed by condensation with triethyl phosphite and hydrolysis of the diethyl ester with trimethylsilyl iodide, to give the - $PO_3H_2$ derivative;
- conversion of the -OH group into a halogen group (for example into a chlorine group by means of thionyl chloride), followed by reaction with potassium thioacetate and subsequent hydrolysis of the acyl group to give the mercapto derivative;
- conversion of the -OH group into an azido derivative (for example by means of Mitsunobu reaction in the presence

of sodium azide, diethylaza dicarboxylate and triphenylphosphine), followed by hydrogenolysis to give the amino derivative;

- alkylation of the -OH group or oft he mercapto group obtained therefrom to give the O-alkyl and S-alkyl derivatives, respectively;
- alkylation of the amino derivative, for example by means of reductive amination, using an aldehyde to generate the alkyl group and sodium cyanoborohydride as a reducing agent, to give the mono- and di-alkylamino derivatives.

It is evident that the above are just examples of suitable reaction patterns. The expert of the art can find several obvious variations of such methods. The reaction conditions for performing all the described methods are also part of the general knowledge of the skilled man and it is not the case to give more details here.

The intermediates of formula (III) are commercial products or can be prepared according to the known methods for synthesizing substituted indoles, first of all the Fischer synthesis. Such a method is part of the general knowledge of the chemist and therefore it is not the case to give more details here.

Compounds of formula (I) in which, in the meanings of A, n is 0 are prepared according to a method which differs from those described above and that encompasses the steps of

(a) transforming a 3,3'-bis(indole)ketone into a 3,3'-bis(indole)methanol by reduction, for example with sodium borohydride;

(b) transforming the hydroxy functionality of the 3,3'-bis(indole)methanol of step (a) into the other substituents as above described for the synthesis of A-CHO.

The 3,3'-bis(indole)ketones are described in J. Heterocyclic Chem., 14(7), 1123-1134 (1977), which is herein incorporated by reference.

The starting materials 3,3'-bis(indole)methanes are obtained by condensation of the indole rings with formaldheyde, as above described for the reaction of A-CHO.

BIOLOGICAL ACTIVITY OF THE COMPOUNDS OF THE INVENTION

Both "in vitro" and "in vivo" tests have been performed.

The compounds of the present invention have been tested in a pharmacological "in vitro" test of inhibition of MMP8 (human neutrophil collagenase). Said test provides for the determination via fluorescence of the inhibition of the degradation of a fluorescent substrate (DNP-Pro-Leu-Gly-Leu-Trp-Ala-D-Arg-$NH_2$, M1855 Bachem) by means of the catalytic domain of MMP8.

*Reagents*:

1) DNP-substrate = DNP- Pro-Leu-Gly-Leu-Trp-Ala-D-Arg-$NH_2$ (M1855 Bachem), M.W. 977,1 g/mol, concentration 25 μM in DMSO; 2) measurement buffer = 50 mM TRIS/100 mM NaCl/10 mM $CaCl_2.2H_2O$, adjusted at pH 7.6 with hydrochloric acid. 3) Enzyme = catalytic domain of MMP8 (92 Kda), concentration 0.055 mg/ml in TRIS buffer. Substrate and enzyme are maintained at 0°C with ice bath.

*Inhibition assay:*

Total volume = 1 ml of solution kept under stirring in a cuvette.

| | |
|---|---|
| Control: | 0.98 ml DMSO |
| | 0.01 ml of DNP-substrate |
| | 0.01 ml of enzyme |
| Assay: | 0.98 ml DMSO |
| | 0.01 ml DNP-substrate |
| | 0.01 ml of enzyme |
| | 0.01 ml of inhibitor (10 μg/ml) |

It is measured the fluorescence at 346 nm both of the control solution (without inhibitor) and of the solution containing the inhibitor. The inhibition of the catalytic activity of MMP8 results in the decrease of the DNP-substrate bond's lysis, with related decrease of the fluorescence of the solution.

The percentage of inhibition is expressed by the following formula:

$$\% \text{ Inhibition} = 100 - (\text{rel. unit/time}_{\text{with inhibitor}}/\text{rel. unit/time}_{\text{control}} \times 100)$$

By repeating the experiment at different concentrations of inhibitor it is possible to determine the $IC_{50}$ value.

The "in vivo" testing was performed against the Lewis Lung (3LL) murine carcinoma according to the following protocol.

Female mice C57BL/6N were inoculated intrafootpad with tumor fragments at day 0. At day 11 the mice were anesthetized and their tumors removed surgically proximal to the popliteale limph nodes. Animals were treated intraperitoneally with the compounds of the invention from one day after surgery until the end of the experiment, day 22 (post-surgery treatment at days 12-15, 18-22). All the compounds were suspended in 0.5% methylcellulose. The mean number of the pulmonary metastases and the mean weight thereof were determined. The toxicity of the compounds of the invention at the administered dosage was also checked, as number of deaths/total no. of treated mice by the end of the experiment.

Table I shows the data for one representative compound of the invention.

Table I - Activity on spontaneous pulmonary metastases deriving from intrafootpad implanted murine Lewis Lung carcinoma (post-surgery treatment, day 12-15, 18-22 after tumor injection)

| compound | dose mg/kg/day | Mean No. of metastases (S.D.) | Mean weight (mg) of metastases (S.D.) | Toxicity |
|---|---|---|---|---|
| 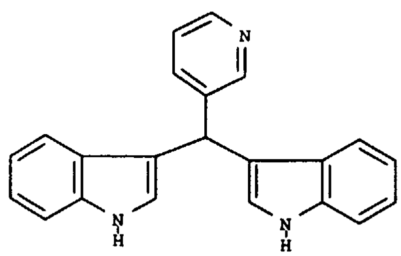 example 1 | 200 | 31.4 (14.0) | 99.2 (63.5) | 0/9 |
| control (no compound administered) | - | 65.9 (11.7) | 197.6 (55.0) | 0/10 |

The compound of the invention showed a significant reduction both in the number and in the weight of the pulmonary metastases when compared to the control animals.

From what it is said above it appears that the compounds of the invention may be used as antitumor and antimetastatic agents to prevent and/or control the progression of primary and secondary tumors in mammals, also in a chronical treatment.

The compounds of the present invention can be administered in doses ranging from 0.01 mg to 0.4 g per kilogram of body weight daily. A preferred dosage regimen to obtain best results is that which provides for the use from about 1 mg to about 50 mg per kilogram of body weight daily, employing unitary doses so that to administer in 24 hours from about 70 mg to about 3.5 g of the active compound to a patient having approximately 70 kg of body weight. Such a dosage regimen may be adjusted to achieve the best therapeutical effect. For example, doses may be administered taking into account the therapeutical situation of the patient. The active compound may be administered by oral, intravenous, intramuscular or subcutaneous route.

The pharmaceutical compositions of the present invention contain therapeutical effective amounts of at least one compound of the invention in admixture with pharmaceutically compatible excipients.

Oral compositions will generally include an inert diluent or an edible carrier. They can be included in gelatin capsules or compressed into tablets. Other oral administration forms are capsules, pills, elixirs, suspensions or syrups.

The tablets, pills, capsules and similar compositions can contain the following ingredients (in addition to the active compound): a binder such as microcrystalline cellulose, tragacanth or gelatin; an excipient such as starch or lactose: a disintegrating agent such as alginic acid, primogel, maize starch and the like; a lubricant such as magnesium stearate;

a fluidifier such as colloidal silicon dioxide; a sweetening agent such as sucrose or saccharine or a flavoring agent such as mint flavor, methyl salicylate or orange flavor.

When the composition selected is in form of capsules, it can contain in addition a liquid carrier such as a fat oil. Other compositions can contain various material which change the physical form thereof for example coating agents (for tablets and pills) such as sugar or shellac. The material used in the preparation of the compositions should be pharmaceutically pure and non toxic at the used dosages.

For the preparation of pharmaceutical compositions for the parenteral administration, the active ingredient can be included in solutions or suspensions, which can comprise in addition the following components: a sterile diluent such as water for injections, saline solution, oils, polyethylene glycols, glycerin, propylene glycol or other synthetic solvents; antibacterial agents such as benzyl alcohol; antioxidants such as ascorbic acid or sodium bisulfite; chelating agents such as ethylenediaminotetracetic acid; buffers such as acetates, citrates or phosphates and agents for adjusting the tonicity of the solution, such as sodium chloride or dextrose. The parenteral preparation can be included in ampoules, mono-dose syringes, glass or plastic vials.

The following examples further illustrate the invention.

<u>Example 1 - 3-[bis(indol-3-yl)methyl]pyridine</u>

To a solution of 3-pyridinecarboxaldehyde (3.2 g) in 21 ml of acetic acid, kept under nitrogen and cooled to 0°C, are added 7 g of indole, adding then further 4 ml of acetic acid. The yellow mixture is kept under stirring for 1 hour, then the reaction mixture is allowed to warm to room temperature. Alter further 3 hours 30 minutes the mixture is concentrated and the residue is partitioned between 100 ml of water and 200 ml of ethyl acetate and it is basified to pH 8-9 by addition of ammonium hydroxide 30%. The organic phase is separated and washed with 75 ml of saturated solution of sodium chloride. The aqueous phase is extracted with further 100 ml of ethyl acetate and the organic extracts are pooled, dried over sodium sulfate and concentrated to dryness. The residue (11.7 g) is recrystallized twice from ethanol (110 ml + 85 ml) and dried under vacuum at 60°C, to give 8.78 g of the product, m.p. 113-115°C.
$^1$H-NMR in d6-DMSO: 5.05 ppm (s, 1H); 6.9 ppm (m; 4H); 7.05 ppm (t, 2H); 7.35 ppm (m, 5H); 7.8 ppm (m, 1H); 8.45 ppm (m, 1H); 8.65 ppm (m, 1H); 10.95 ppm (s, 2H).

<u>Example 2</u>

According to the method described in example 1, starting from the suitable intermediates, the following bis-indoles are prepared:

(1) 3-[bis(5-hydroxyindol-3-yl)methyl]pyridine,
$^1$H-NMR in d6-DMSO: 5.65 ppm (s, 1H); 6.55 ppm (d, 2H); 6.6 ppm (s, 2H); 6.7 ppm (d, 2H); 7.1 ppm (d, 2H); 7.25 ppm (m, 1H); 7.6 ppm (m, 1H); 8.35 ppm (m, 1H); 8.5 ppm (s, 2H); 8.55 ppm (m, 1H); 10.55 ppm (s, 2H);
(2) 2-[bis(indol-3-yl)methyl]pyridine;
(3) 4-[bis(indol-3-yl)methyl]pyridine;
(4) 3-[bis(indol-3-yl)methyl]quinoline;
(5) 2-[bis(5-hydroxyindol-3-yl)methyl]quinoline;
(6) 8-[bis(5-methylindol-3-yl)methyl]quinoline;
(7) 4-[bis(5-methoxyindol-3-yl)methyl]isoquinoline;
(8) 2-[bis(6-acetoxyindol-3-yl)methyl]thiophene;
(9) 2-[bis(indol-3-yl)methyl]thiophene;
(10) 2-[bis(indol-3-yl)methyl]imidazole;
(11) 2-[bis(5-fluoroindol-3-yl)methyl]imidazole;
(12) 3-[bis(6-chloroindol-3-yl)methyl]pyrrole;
(13) 2-[bis(5-hydroxyindol-3-yl)methyl]furane;
(14) 5-methyl-2-[bis(indol-3-yl)methyl]furane;
(15) 5-methyl-2-[bis(5-mercaptothdol-3-yl)methyl]furane;
(16) 5-hydroxymethyl-2-[bis(indol-3-yl)methyl]furane;
(17) 8-hydroxy-2-[bis(5-hydroxyindol-3-yl)methyl]quinoline;
(18) 8-hydroxy-2-[bis(indol-3-yl)methyl]quinoline;
(19) 8-hydroxy-2-[bis(6-methoxycarbonylindol-3-yl)methyl]quinoline;
(20) 6-(pyridin-2-yl)-3-[bis(indol-3-yl)methyl]pyridine;
(21) 2-carboxy-1-[bis(5-hydroxyindol-3-yl)methyl]benzene;
(22) 3-carboxy-4-hydroxy-1-[bis(indol-3-yl)methyl]benzene;
(23) 4-methoxycarbonyl-1-[bis(5-aminoindol-3-yl)methyl]benzene;

(24) 3,4-dihydroxy-1-[bis(6-methylaminoindol-3-yl)methyl]benzene;
(25) 2,3-dihydroxy-1-[bis(6-diethylaminoindol-3-yl)methyl]benzene;
(26) 2,3-dihydroxy-1-[bis(indol-3-yl)methyl]benzene;
(27) 3-carboxy-1-[bis(6-ethylthioindol-3-yl)methyl]benzene;
(28) 4-carboxy-1-[bis(5-bromoindol-3-yl)methyl]benzene;
(29) 3,4-dihydroxy-1-[bis(5-isopropylindol-3-yl)methyl]benzene;
(30) 2-mercapto-1-[bis(5-hydroxyindol-3-yl)methyl]benzene;
(31) 3-hydroxyaminocarbonyl-1-[bis(indol-3-yl)methyl]benzene;
(32) 1-[bis(5-iodoindol-3-yl)methyl]benzene-3-phosphonic acid;
(33) 2-[bis(5-hydroxyindol-3-yl)methyl]acetic acid;
(34) 2-[bis(6-methylthioindol-3-yl)methyl]acetic acid;
(35) 2-[bis(5-n-butylindol-3-yl)methyl]acetic acid;
(36) 3-[bis(6-methoxyindol-3-yl)methyl]propionic acid;
(37) 3-[bis(indol-3-yl)methyl]propan-1-thiol;
(38) 2-[bis(5-hydroxyindol-3-yl)methyl]ethane-1-phosphonic acid;
(39) 2,2-[bis(5-hydroxyindol-3-yl)]acetic acid;
(40) 2-[bis(5-hydroxyindol-3-yl)methyl]-1-aminoethane;
(41) 3-[bis(6-ethylindol-3-yl)methyl]-1-aminopropane;
(42) 2-[bis(5-ethoxycarbonylindol-3-yl)methyl]-1-dimethylaminoethane;
(43) 2-[bis(indol-3-yl)methyl]ethane-1-hydroxamicacid;
(44) 2-[bis(2-ethylindol-3-yl)methyl]acetic acid;
(45) 2,3-dihydroxy-1-[bis(2-hydroxyindol-3-yl)methyl]benzene;
(46) 2-[bis(2-aminoindol-3-yl)methyl]pyridine;
(47) 3-[bis(2-methylindol-3-yl)methyl]quinoline;
(48) 1-[bis(2-hydroxymethylindol-3-yl)methyl]isoquinoline;
(49) 2-[bis(2-isopropylindol-3-yl)methyl]imidazole.

## Claims

**1.** The use of the compounds of the general formula (I):

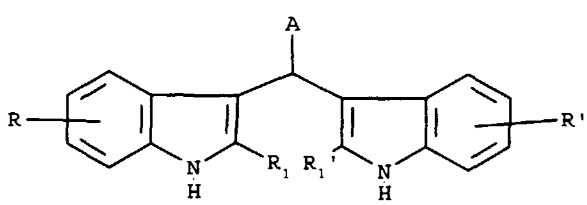

(I)

wherein:

- R and R' are independently selected from hydrogen, hydroxy, chlorine, bromine, iodine, fluorine, $(C_1-C_6)$alkyl, $(C_1-C_4)$alkoxy, $(C_1-C_4)$acyloxy, amino, mono-$(C_1-C_4)$alkylamino, di-$(C_1-C_4)$alkylamino, -SH, $(C_1-C_4)$alkylthio, carboxy, $(C_1-C_4)$alkoxycarbonyl groups;
- $R_1$ and $R_1$' are independently selected from hydrogen, hydroxy, hydroxymethyl, amino, carboxy, $(C_1-C_4)$alkyl groups;
- A is a
    - phenyl or naphtyl group substituted by at least one group selected from hydoxy, carboxy, -SH, -CONHOH or $-PO_3H_2$ group, or is a
    - 5- or 6-membered heterocycle containing one or two heteroatoms selected from oxygen, nitrogen or sulfur, which can be optionally benzocondensed and/or substituted by at least one group selected from pyridyl, -SH, $-PO_3H_2$, carboxy or

- CONHOH, or is a

    - group of formula $-(CH_2)_n-X$, in which n is zero or the integers 1 or 2, and X is selected from -SH, $-PO_3H_2$,

-CONHOH, carboxy, amino, mono-($C_1$-$C_4$)alkylamino, di-($C_1$- $C_4$)alkylamino, enantiomers, diastereoisomers, racemates and mixtures thereof, as well as salts thereof with pharmaceutically acceptable acids and bases, for the preparation of a medicament having antitumor and antimetastatic activity.

**2.** The use according to claim 1, wherein the compound is 3-[bis(indol-3-yl)methyl]pyridine or 3-[bis(5-hydroxyindol-3-yl)methyl]pyridine.

**3.** Compounds according to the general formula (I) of claim 1, provided that the following compounds are disclaimed:

- compounds in which R, R', $R_1$, $R_1$' are hydrogen and A is pyridyl 2-furanyl, 2-thienyl, carboxy, methoxycarbonyl, ($C_1$-$C_4$)alkoxycarbonylmethyl, 2- or 4-hydroxyphenyl, 3,4-dihydroxyphenyl;
- $R_1$, $R_1$' are hydrogen, R and R' are 6-bromo and A is a aminomethyl group;
- $R_1$, R,' are hydrogen, R and R' are 5-chloro, 5-bromo, 5-carboxy or 5-methoxy and A is a 4-pyridyl group;
- R, R' are hydrogen, $R_1$ and $R_1$' are methyl and A is 2-furanyl, 2-thienyl or 2-hydroxyphenyl.

**4.** Compounds according to claim 3, in which A is selected from pyridyl, imidazol-2-yl, thienyl, quinolinyl, isoquinolinyl, pyrrolyl, furanyl, 5-methylfuran-2-yl, 5-hydroxymethylfuran-2-yl, 8-hydroxyquinolin-2-yl, dipyridyl.

**5.** Compounds according to claim 3, in which A is selected from 2-carboxyphenyl, 3-carboxy-4-hydroxyphenyl, 4-methoxycarbonylphenyl, 3,4-dihydroxyphenyl, 2,3-dihydroxyphenyl, 3-carboxyphenyl, 4-carboxyphenyl.

**6.** Compounds according to claims 3 to 5, in which $R_1$ and $R_1$ and/or R and R' are hydrogen.

**7.** Compounds according to claims 3 to 6, in which A is a 2-,3- or 4-pyridyl group.

**8.** A compound according to claim 3, selected from:

- 3-[bis(5-hydroxyindol-3-yl)methyl]pyridine,
- 3-[bis(indol-3-yl)methyl]quinoline;
- 2-[bis(5-hydroxyindol-3-yl)methyl]quinoline;
- 8-[bis(5-methylindol-3-yl)methyl]quinoline;
- 4-[bis(5-methoxyindol-3-yl)methyl]isoquinoline;
- 2-[bis(6-acetoxyindol-3-yl)methyl]thiophene;
- 2-[bis(indol-3-yl)methyl]imidazole;
- 2-[bis(5-fluoroindol-3-yl)methyl]imidazole;
- 3-[bis(6-chloroindol-3-yl)methyl]pyrrole;
- 2-[bis(5-hydroxyindol-3-yl)methyl]furane;
- 5-methyl-2-[bis(indol-3-yl)methyl]furane;
- 5-methyl-2-[bis(5-mercaptoindol-3-yl)methyl]furane;
- 5-hydroxymethyl-2[bis(indol-3-yl)methyl]furane;
- 8-hydroxy-2-[bis(5-hydroxyindol-3-yl)methyl]quinoline;
- 8-hydroxy-2-[bis(indol-3-yl)methyl]quinoline;
- 8-hydroxy-2-[bis(6-methoxycarbonylindol-3-yl)methyl]quinoline;
- 6-(pyridin-2-yl)-3-[bis(indol-3-yl)methyl]pyridine;
- 2-carboxy-1-[bis(5-hydroxyindol-3-yl)methyl]benzene;
- 3-carboxy-4-hydroxy-1-[bis(indol-3-yl)methyl]benzene;
- 4-methoxycarbonyl-1-[bis(5-aminoindol-3-yl)methyl]benzene;
- 3,4-dihydroxy-1-[bis(6-methylaminoindol-3-yl)methyl]benzene;
- 2,3-dihydroxy-1-[bis(6-diethylaminoindol-3-yl)methyl]benzene;
- 2,3-dihydroxy-1-[bis(indol-3-yl)methyl]benzene;
- 3-carboxy-1-[bis(6-ethylthioindol-3-yl)methyl]benzene;
- 4-carboxy-1-[bis(5-bromoindol-3-yl)methyl]benzene;
- 3,4-dihydroxy-1-[bis(5-isopropylindol-3-yl)methyl]benzene;
- 2-mercapto-1-[bis(5-hydroxyindol-3-yl)methyl]benzene;
- 3-hydroxyaminocarbonyl-1-[bis(indol-3-yl)methyl]benzene;
- 1-[bis(5-iodoindol-3-yl)methyl]benzene-3-phosphonic acid;
- 2-[bis(5-hydroxyindol-3-yl)methyl]acetic acid;
- 2-[bis(6-methylthioindol-3-yl)methyl]acetic acid;

- 2-[bis(5-n-butylindol-3-yl)methyl]acetic acid;
- 3-[bis(6-methoxyindol-3-yl)methyl]propionic acid;
- 3-[bis(indol-3-yl)methyl]propan-1-thiol;
- 2-[bis(5-hydroxyindol-3-yl)methyl]ethane-1-phosphonic acid;
- 2,2-[bis(5-hydroxyindol-3-yl)]acetic acid;
- 2-[bis(5-hydroxyindol-3-yl)methyl]-1-aminoethane;
- 3-[bis(6-ethylindol-3-yl)methyl]-1-aminopropane;
- 2-[bis(5-ethoxycarbonylindol-3-yl)methyl]-1-dimethylaminoethane;
- 2-[bis(indol-3-yl)methyl]ethane-1-hydroxamic acid;
- 2-[bis(2-ethylindol-3-yl)methyl]acetic acid;
- 2,3-dihydroxy-1-[bis(2-hydroxyindol-3-yl)methyl]benzene;
- 2-[bis(2-aminoindol-3-yl)methyl]pyridine;
- 3-[bis(2-methylindol-3-yl)methyl]quinoline;
- 1-[bis(2-hydroxymethylindol-3-yl)methyl]isoquinoline;
- 2-[bis(2-isopropylindol-3-yl)methyl]imidazole.

**9.** Pharmaceutical compositions containing an effective dosage of at least one compound according to claims 3 to 8 in admixture with pharmaceutically acceptable excipients or diluents.

European Patent Office

# EUROPEAN SEARCH REPORT

Application Number
EP 97 11 0336

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | STRELL ET AL.: "Die Umsetzung von Pyridin- und Chinolinaldehyden mit Pyrrolen und Indolen" CHEMISCHE BERICHTE, vol. 90, no. 9, 1957, pages 1798-1808, XP002046948 * examples 12-14,17 * --- | 3-8 | C07D401/14<br>A61K31/40<br>C07D409/14<br>C07D403/14<br>C07D405/14<br>C07D209/10<br>C07F9/38 |
| X | M. JULIA; G. TILLY: "Recherches en série indolique. XV.-Sur l'acide di(indolyl-3)acētique et ses esters et quelques di(indolyl-3)êthyl (et propyl) amines" BULLETIN DE LA SOCIETE CHIMIQUE DE FRANCE, vol. 8, August 1965, pages 2175-2181, XP002046949 * examples 2,4,7 * --- | 3-9 | |
| X | C.W. REES; C.R. SABET: "Mechanism of the reaction of phthalaldehydic acid with indoles. Intramolecular catalysis in aldehyde reactions" JOURNAL OF THE CHEMICAL SOCIETY, January 1965, pages 680-687, XP002046950 * page 683; example XV * --- | 3-8 | TECHNICAL FIELDS SEARCHED (Int.Cl.6)<br>C07D<br>A61K<br>C07F |
| X | A. CALVAIRE; R. PALLAUD: "Sur la préparation de quelques dérivés indoliques" COMPTES RENDUS HEBDOMADAIRES DES SEANCES DE L'ACADEMIE DES SCIENCES, vol. 258, 1964, pages 609-612, XP002046951 * page 611-612; examples XVIII,XX * --- | 3-8 | |
| | -/-- | | |

The present search report has been drawn up for all claims

| Place of search | Date of completion of the search | Examiner |
|---|---|---|
| MUNICH | 14 November 1997 | Lauro, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 97 11 0336

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | M. RIDI: "Alcune reazioni sopra derivati del pirazolone" GAZZETTA CHIMICA ITALIANA, vol. 71, 1941, pages 542-546, XP002046952 * example V * | 3-8 | |
| X | S.H. ZEE; C.S. CHEN: "The reaction of indole with propiolic acid" JOURNAL OF THE CHINESE CHEMICAL SOCIETY, vol. 21, no. 4, 1974, pages 229-234, XP002046953 * see compound IV * | 3-8 | |
| A | WO 97 07097 A (AUCKLAND DIVISION CANCER SOCIETY OF NEW ZEALAND INC.) * page 13; claim 1 * | 1-9 | |
| A | US 4 866 084 A (GUNASEKERA ET AL.) * column 1-2 * | 1-9 | TECHNICAL FIELDS SEARCHED (Int.Cl.6) |
| A | US 5 516 915 A (BARTH ET AL.) * column 1-2 * | 1-9 | |

The present search report has been drawn up for all claims

| Place of search | Date of completion of the search | Examiner |
|---|---|---|
| MUNICH | 14 November 1997 | Lauro, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)